# EUROPEAN PATENT SPECIFICATION

(11) **EP 1 263 485 B1**
(45) Date of publication and mention of the grant of the patent: **02.03.2005**
(21) Application number: 00909347.7
(22) Date of filing: 09.03.2000
(51) Int. Cl.: A61L 27/24, A61L 27/44, A61L 27/50, A61L 15/32

(54) **MULTILAYER COLLAGEN MATRIX FOR TISSUE RECONSTRUCTION**
MEHRSCHICHT-KOLLAGENMATRIX ZUR GEWEBEREKONSTRUKTION
MATRICE COLLAGENE MULTICOUCHES DESTINEE A LA RECONSTRUCTION DES TISSUS

(43) Date of publication of application: 11.12.2002
(73) Proprietor: Syntacoll AG, 9100 Herisau (CH)
(72) Inventor: RUSZCZAK, Zbigniew, D-81925 München (DE); MEHRL, Robert, D-84085 Langquaid (DE); JECKLE, Johann, D-93339 Riedenburg (DE); STOLTZ, Michael, D-81827 München (DE)
(74) Representative: Böhm, Brigitte, Dipl.-Chem. Dr.
(86) International application number: PCT/EP2000/002055
(87) International publication number: WO 2001/066162

(56) References cited:
- WO-A-98/22158
- WO-A-99/19005
- US-A- 4 522 753
- US-A- 4 948 540
- US-A- 5 206 028
- US-A- 5 219 576
- US-A- 5 733 337
- US-A- 5 863 984

## Description

The invention relates to a novel multilayer material with improved mechanical, physical, functional and handling properties for use in human and veterinary medicine in both in vivo and in vitro condition and/or for in ex-vivo and/or in in vivo reconstruction of tissue or organs, as well as to a process for the manufacture of such novel material.

The use of various different xenogenous, allogenic or autologous collagen-based materials in human and veterinary medicine is known. Such collagen materials can be used for example as hemostatic agent, as a substitute of missing tissue, as a skin equivalent, as a material for tissue augmentation or as a carrier for biologically active substances.

Purified collagen, even of xenogenous origin, is almost fully biocompatible with human (and also animal of different species) collagenous tissue and may be incorporated into and/or subsequently remodeled to a host connective tissue without foreign body reaction and immunologic rejection.

If used as a hemostatic agent, collagen-based material must have both biological and mechanical features promoting hemostasis such as intact collagen fibers and optimal porosity.
For use as tissue substitute (equivalent) the collagen-based material must have optimal matrix properties promoting cell growth, formation of granulation tissue, angiogenesis and vascularization.

As carrier of biologically active substances the collagen-based material must have features allowing an optimal release and pharmacokinetic of incorporated substances.
In all cases, however, the handling of the collagen-based material, its mechanical stability, flexibility and, if necessary, its ability to be sutured or sealed are still most important.

The most popular commercially available collagen-based materials are sponges, membranes or injectable solutions of different viscosity.

For tissue substitution, hemostasis, skin substitution and as a carrier for biologically active substances both collagen-based sponges and membranes have been used in both experimental and clinical studies.
However, there are only few collagen-based drug carriers available on the international market. For example, the only one commercially available collagen-based drug delivery system (for antibiotics) is Collatamp® -G (manufacturer: SYNTACOLL AG, Herisau, Switzerland), also known under 18 different trade names and distributed worldwide by Schering-Plough (USA) and its subsidiaries.

All currently available collagen-based materials are, however, not stable enough to be sutured, rolled, or sticked, especially in areas of mechanical tension or in difficult anatomical sites.
Moreover, collagen sponges or membranes are - in many cases - not strong enough to sufficiently cover defects of such tissue as, i.e., dura mater, superficial and deep skin wounds, bones, nerves, etc.,

To improve the mechanical properties of collagen materials, various additional crosslinking procedures have been described. The most popular are: chemical crosslinking (i.e. with aldehydes) or physical crosslinking (i.e. dehydro-thermal treatment).

The aldehyde-based crosslinking may negatively influence the biocompatibility of collagen and lead to some residues of aldehydes (or its derivatives) in the final product.
The dehydro-thermal treatment, which is used mostly for collagen sponges, has its natural limitation and does not lead to products with the desired properties.

US-Patent 4,655,980 describes the possible manufacturing of a collagen membrane based on a soluble collagen gel suspension. The membrane may be obtained by applying pressure to the gel, or by disrupting the gel and separating the resulting precipitate for casting. Depending on the dimension and shape of the casting mold, either a membrane or solid can be obtained. In fact, the manufacturing of such membrane is based on a commercially available soluble, injectable, atelocollagen product of Collagen Aesthetics, Palo Alto, CA, USA.

The US-Patent 5,219,576 describes a collagen implant material useful as wound healing matrices and delivery system for bioactive agents. Besides manufacturing traditional collagen sponges based on casting and drying of a soluble collagen gel suspension, the patent describes the manufacturing of multilayer material by casting and freezing the individual layers and then lyophilizing the entire composite at once. A possibility of additional crosslinking by both aldehyde and dehydro-thermal processing of the final product is also discussed.

The US-Patent 4,522,753 describes, inter alia, a method for preserving porosity and improving stability of collagen sponges by both aldehyde and dehydro-thermal treatment. The negative pressure (vacuum) used may vary from about 1 mtorr up to slight vacuum just below atmospheric pressure.

The US-Patent 4,578,067 describes a hemostatic-adhesive collagen dressing in form of dry-laid, non-woven, self-supporting webs of collagen fibers. The manufacturing of such material is based on a Rando-feeder and Rando-webber techniques. The collagen fibers from the Rando-feeder are introduced into the air stream of the Rando-webber and form a fiber mass of uniform density. Such mass may then be processed by pressing or embossing or by calendering at a temperature ranging from room temperature to 95°C.

The US-Patent 5,206,028 describes a collagen membrane having improved physical and biological properties. Such membrane does not swell appreciably upon being wetted and maintains its density. The manufacturing of such translucent, collagen Type-I based material is based on compression of collagen sponges in a roller press with a calibrate aperture followed by aldehyde cross-linking. For additional mechanical stabilization, the cross-linked membrane may be re-wetted, re-lyophilized and pressed again under standard condition.

The US-Patent 4,948,540 describes a mechanically stable, comfortable collagen wound dressing sheet material fabricated by lyophilizing a collagen composition (soluble and insoluble collagen parts in range of 1:20 to 10:1) and compressing the porous pad at a pressure between about 15,000 and 30,000 p.s.i. The material may be also cross-linking by dehydro-thermal treatment to improve mechanical stability.

At present, all of the methods for manufacturing of collagen-based material with improved mechanical, physical and biological properties (as described above) are not in use for industrial manufacturing of such collagen-based material.

There is a need, in both human and veterinary medicine, to create collagen-based materials (or materials based on other natural polymers) with enhanced mechanical and physical properties without increasing immunologic disadvantages as well as without potential risk for foreign body reaction or granuloma formation.

Moreover, there is a need to create collagen-based constructs in which collagen components can be joined on physical and/or mechanical basis without additional cross-linking substances of potential negative value for living cells or tissue.
Additionally, there is a need to create such collagen-based constructs which can serve for long time tissue substitution (slow degradation or/and incorporation rate) and have high mechanical strength.
There is also a need to create such collagen-based materials which are multilayered and in which each layer can have different mechanical or physical and physiologic properties, incl. wetting time, absorption time and/or remodeling/degradation time.
This is of particular interest, if the construct is meant to be used as a matrix for organ substitution or as a delivery system for biologically active substances or drugs.

It was, therefore, an object of the present invention, to provide collagen-based materials which satisfy the above needs and avoid the drawbacks of the state of the art materials.

This object is solved according to the invention by a multilayer material with improved mechanical, physical, funtional and handling properties for use in human and veterinary medicine in both in vivo and in vitro condition and/or for in ex vivo and/or in vivo reconstruction of tissue organs wherein the material
comprises layers of
a) at least one material comprising a natural polymer collagen of animal or/and human or/and recombinant or/and transgenic (incl similar or adequate techniques) origin
   and/or
b) at least one material comprising a an optionally biocompatible collagen,
and wherein the material is obtainable by joining the layers by simultaneous treatment with defined heat and defined pressure.

Within the context of this invention, natural polymer is meant to encompass natural substances which exhibit similar properties as collagen and are useful for the same applications. Examples of such substances are collagen, gelatine and hyaluronic acid. According to the invention collagen is the preferred natural polymer.

This invention allows to permanently or temporarily join different natural polymer- and, preferably, collagen-containing products or layers under formation of a multilayered product
by simultaneously applying defined mechanical pressure and defined heat - to at least two preferably different product layers - in a way which protects the fibrilar - native and/or renaturate - structure of the collagen from degradation or/and denaturation or/and melting and which save the natural biologic properties of collagen (i.e. hemostatic properties or matrix properties).
Moreover, the method used allows to create natural polymer- and, preferably, collagen-based material with improved and variable, but defined, mechanical stability, dry and wet tension, fluid absorption and flexibility.

The present invention allows the joining of at least two layers of the same materials. Such layers are all natural polymer, collagen based. The final product - especially in the case of two collagen-based layers in the material - has excellent mechanical properties, especially dry and wet tension. Moreover such material has natural hemostatic properties, improved wetting abilities, and can be rolled or screwed in dry or wet condition without loosing shape, braking down, etc.

The present invention allows for example the combination (joining) of different collagen fashions such freeze-dried sponges, air-dried membranes, freeze-dried pre-pressed sponges, etc,.

The present invention allows to create novel collagen-based materials such, i.e., leather-like collagen sheets of different strength , collagen "pockets" or "tortellini-like" constructs, collagen "sandwich"-like structures of different permeability and porosity as well as collagen tubes and channels with or without lumen. In the latter case, the "lumen" of the tube (channel) may be filled with other collagen-based material (core) of various density and/or porosity.

The collagen used for manufacture of the improved collagen-based multilayer material of the invention may be either of animal origin (xenogenous to humans) or human origin (autologous or allogenic) or may be obtained from genetically manipulated organisms (recombinant techniques and/or transgenic organisms), or by any other similar or/and equivalent method.

Moreover, due to variations in the manufacturing process, the permeability for air (or other gases) and water (or other fluids, incl. blood, tissue fluids or similar) as well as the mechanical strength of the final product can easily be controlled. The respective methods are basically known to the skilled artisan.

The collagen used for manufacturing of the improved collagen-based material may be of Typ-I, Type-II, Type-III, Type-IV, Type-VII, Type-IX alone or may be a mixture of two or more of such collagens.

As the most important collagen in the human and animal body is the Typ-I collagen, the raw material preferentially used for manufacture of such improved collagen-based multilayer product is the Type-I collagen. This material can be easily obtained i.e. from animal tissue (skin, tendons, etc.,) by industrial methods according to state-of-the-art, GMP-conformed techniques.
Both enzymatically treated or not enzymatically treated collagen can be used for manufacture. If treated with proteolytic enzymes, non-helical parts of the collagen molecule will be separated from the triple-helical collagen chain (atelocollagen).

To obtain the novel multilayer membrane-like material of appropriate mechanical and physiological properties, different layers a natural polymer, preferentially pre-pressed collagen membranes, non-pre-pressed collagen sponges or air-dried collagen membranes alone or in different combinations may be used.

The influence of a moderate heat, especially if used together with a negative pressure (vacuum), for induction of additional cross-linking sites in collagen sponges has been described previously as dehydro-thermal treatment (see above).

The present invention now combines heat and positive pressure (mechanical pressure), both known per se to the skilled worker, for the treatment of the basic materials according to the present invention. Such combination has never been proposed before, but leads to products with highly unexpected, superior properties, as described above.

The temperature used lies preferably in a range of from 50°C to 200°C.
The pressure used lies preferably in a range of from 0.1 to 1000 kg/cm².
The time period of the thermal pressing procedure lies preferably between 0.1 second to 1 hour.
The treatment can be conducted in a conventional thermal pressing machine in which the parts exerting the pressure can be adjusted to a predefined and constant temperature. The manufacturing steps used for the preparation of the novel multilayer material can be easily incorporated into routine manufacturing process and allow to save time and costs if compared to other currently used methods used for the production of collagen membranes etc.

As a result of such a heat and pressure treatment, a collagen-containing membrane-like structure of desired thickness, mechanical strength, permeability, degradation and resorption time, etc., can be manufactured.

Moreover, the manufactured product is much better in handling than other known collagen based products such as freeze-dried sponges or air-dried membranes.

The basis material for manufacturing the novel multilayer material is preferably a pre-pressed, non-transparent collagen membrane, non pre-pressed collagen sponge, a transparent collagen membrane or a combination of these products.

The collagen sponge may be manufactured using various state-of-the-art techniques. The basis for such a material may be collagen dispersion / suspension (i.e. in water or other non-organic solvent) of 0.5 to 5.0 weight% of dry collagen.
The sponge can obtained preferably by freeze-drying.

To improve basic mechanical properties, the collagen sponge itself can preferably be treated simultaneously with defined heat and defined pressure, to obtain a non-transparent, membrane-like structure.
The temperature used for such treatment again preferably lies within a range of from 50°C to 200°C.
The pressure used for such treatment also again lies in a range of from 0.1 to 1000 kg/cm².
The time period for the thermal pressing procedure is preferably set between 0.1 seconds and 1 hour.

A transparent collagen membrane may be manufactured using different state-of-the-art techniques. The basis for such a material may also be collagen dispersion / suspension (i.e. in water or other non-organic solvent) of 0.5 to 5.0 weight% of dry collagen.
The membrane will preferably be obtained by controlled air-drying.
Such preformed membranes, preferably, can be used as one or more of the layers on collagen basis.

The material used for manufacturing such multilayer material according to the invention may be a natural polymer or/and artificial (synthetic) polymer in various different structure. This allows for the combination of either only natural polymers of the same or different kind, as well as the combination of (optionally several) natural polymers and synthetic polymers to form a multilayer structure of the invention and useful for the above mentioned applications and indications.

Both the natural polymer material and the synthetic polymer material optionally may contain further substances like biologically active substances such as hemostatic agents, growth factors, cytokines, hormones, drugs (i.e. antibiotics, antiinflammatory agents or the like), etc. or biologically important and tissue-compatible inorganic or/and organic substances or/and their derivatives which can improve the mechanical, functional, biological and handling properties of the material.

Another subject of the present invention is the process for the manufacture of the multilayer material of the invention, as already described above in detail as to basic materials used and process conditions.

Still another subject of the present invention is the use of the novel multilayer material of the invention for the indications and applications mentioned above in the context of the description of the material.

The invention will be further described and illustrated by the following examples.

### EXAMPLES

Note: using different amounts of basic collagen sponges or basic collagen membranes both mechanical properties and biological function (especially remodeling/degradation ratio) of the final material can be influenced. Moreover, using different forms of basic material (non-preprocessed or pre-processed) as well as various different ingredients (i.e. biologically active substances) incorporated into the basic material, the degradation ratio and release ratio may be influenced (controlled) in various different ways.

In these examples the manufacturing of the simplest material made from two or three similar or different basic materials (sponges or/and membranes) will be described.

### Example 1

### Manufacture of a novel multilayer collagen-based membrane-like material from two (or more) basic, not pre-pressed sponges.

Two freeze-dried collagen sponges (i.e. Colfatamp®, manufacturer: SYNTACOLL AG, Herisau, Switzerland) will be conditioned at 21 °C in a moisture chamber to a water content of 14%. After conditioning, the sponges are placed one above the other to form a double-layer and prepared for thermo-mechanical pressing.
The continuous heat and pressure, 100°C and 25 kg/cm² respectively, is applied to the sponges for 10 seconds to form a double-layer construct.
After pressing, the surfaces of the press are opened without pre-cooling. The collagen-based double-layer membrane obtained is not transparent. It has an excellent mechanical stability, flexibility, good fluid absorption and good hemostatic properties.
The collagen layers are joined physically, the mechanical stability of the junction is very high.
The material obtained can be used in various medical applicationd in both ex vivo and in vivo situation.

### Example 2

### Manufacture of a novel multilayer collagen-based membrane-like material (pre-pressed sponges)

Two freeze-dried collagen sponges (i.e. Collatamp®, manufacturer: SYNTACOLL AG, Herisau, Switzerland) will be pressed to a double-layer membrane as in Example 1. After pressing, the membranes are conditioned to a water content of 10-15% and placed one above the other to form a double-layer and prepared for thermo-mechanical pressing.
The continuous heat and pressure, 100°C and 25 kg/cm² respectively, is applied to the sponges for 10 seconds to form a new double-layer construct. Each layer of this material consists of two previously pressed sponges of defined porosity.
After finishing pressing, the surfaces of the press are opened without pre-cooling. The collagen-based double-layer membrane obtained is not transparent. It has an excellent mechanical stability, flexibility, good hemostatic properties, but only limited fluid absorption due to the very low porosity.
The collagen layers are joined physically, the mechanical stability of the junction is very high.
The material obtained can be used in various medical applications in both ex vivo and in vivo situation.

### Example 3

### Manufacture of a novel multilayer collagen-based membrane-like material (not pre-pressed sponges + pre-pressed sponges)

Two freeze-dried collagen sponges (i.e. Collatamp®, manufacturer: SYNTACOLL AG, Herisau, Switzerland) will be conditioned at 21°C in a moisture chamber to a water content of 14%. After conditioning, the sponges are placed one above the other to form a double-layer and prepared for thermo-mechanical pressing. This will lead to a double-layer construct as in Example 1.

On the top of this material, an additional freeze-dried collagen sponge (i.e. Collatamp® , manufacturer: SYNTACOLL AG, Herisau, Switzerland) will be placed. In such case, two layers of this material consist of two previously pressed sponges, the third one of a not-pressed sponge of defined porosity. This three-layer construct is then pressed to a membrane. A continuous heat and pressure, 100°C and 25 kg/cm², respectively, is applied to the sponges for 10 seconds to form a new three-layer construct.
After finishing pressing, the pressure surfaces are opened without pre-cooling. The collagen-based double-layer membrane obtained is not transparent. It has an excellent mechanical stability, flexibility and good hemostatic properties. The previously pre-pressed layer has a very limited fluid absorption, but the third (spongy) layer has an excellent fluid absorption and can absorb a fluid amount of up to 10 times of the own weight.
All collagen layers are joined physically, the mechanical stability of the junction is very high.
The material obtained can be used in various medical applications in both ex vivo and in vivo situation, especially as a wound covering material, hemostatic material, etc.

### Example 4

### Manufacture of a novel multilayer collagen-based membrane-like material (not pre-pressed sponges + air-dried membrane)

A freeze-dried collagen sponge (i.e. Collatamp®, manufacturer: SYNTACOLL AG, Herisau, Switzerland) is conditioned at 21 °C in a moisture chamber to a water content of 14%.
On the top of this sponge, an air-dried transparent collagen membrane (i.e. Collatamp-Fascia®, manufacturer: SYNTACOLL AG, Herisau, Switzerland) will be placed. The Fascia is conditioned to a water content of 20%. This double-layer construct is then pressed to a membrane. A continuous heat and pressure, 100°C and 25 kg/cm², respectively, is applied to the sponges for 10 seconds to form a new double-layer construct.
After finishing pressing, the surfaces or the press are opened without pre-cooling. The collagen-based double-membrane obtained is not transparent. It has an excellent mechanical stability, flexibility and good hemostatic properties. The previously spongy layer has an excellent fluid absorption and can absorb a fluid amount of up to 10 times of the own weight. The previously fascia layer remains hemostatic, but absorbs fluids in only limited quantity. This layer serves both as mechanical and biological barrier of limited water and air permeability covering the surface of the product.
Both collagen parts are joined physically, the mechanical stability of the junction is very high.
The material obtained here can be used in various medical applications in both ex vivo and in vivo situation, especially as a wound covering material, hemostatic material, dressing for split- of full-skin donor sites, etc.

### Example 5

### Manufacture of a novel multilayer collagen-based membrane-like material (pre-pressed sponges + air-dried membrane)

Two freeze-dried collagen sponges (i.e. Collatamp®, manufacturer: SYNTACOLL AG, Herisau, Switzerland) will be conditioned at 21 °C in a moisture camber to a water content of 14%. After conditioning, the sponges are placed one above the other to form a double-layer and prepared for thermo-mechanical pressing. This will lead to a double-layer construct as in Example 1.
On the top of this material, an air-dried transparent collagen membrane (i.e. Collatamp-Fascia®, manufacturer: SYNTACOLL AG, Herisau, Switzerland) will be placed. The Fascia is conditioned to a water content of 20%. In such case, two layers of this material consist of two previously pressed sponges, the third one of a not-pressed but flexible collagen mambrane. This three-layer construct is then pressed to a membrane. A continuous heat and pressure, 100°C and 25 kg/cm², respectively, is applied to the sponges for 10 seconds to form a new three-layer construct.
After finishing pressing, the press surfaces are opened without pre-cooling. The collagen-based triple-layer membrane obtained is not transparent. It has an excellent mechanical stability, flexibility and good hemostatic properties. The previously spongy layer has lower fluid absorption than previously not pre-pressed one, and can absorb a fluid amount of up to 10 times of the own weight. The previously fascia layer remains hemostatic, but absorbs fluids in only limited quantity. This layer serves both as mechanical and biological barrier of limited water and air permeability covering the surface of the product.
Both collagen parts are joined physically, the mechanical stability of the junction is very high.
The material obtained here can be used in various medical applications in both ex vivo and in vivo situation, especially as a wound covering material, hemostatic material, dressing for split- of full-skin donor sites, etc.

### Example 6

### Manufacture of a novel multilayer collagen-based material in form of tubes of channels with open lumen.

Two freeze-dried collagen sponges (i.e. Collatamp®, manufacturer: SYNTACOLL AG, Herisau, Switzerland) will be conditioned at 21 °C in a moisture chamber to a water content of 14%. After conditioning, the sponges are placed one above the other to form a double-layer and prepared for thermo-mechanical pressing. This will lead to a double-layer construct as in Example 1.

This construct will be then turned around a tube made from not adhesive, thermo-stable agent (i.e. medical grade paper) and thermally pressed to obtain a collagen-membrane tube. A continuous heat and pressure, 100°C and 25 kg/cm², respectively, is applied to a period of sponges for 10 seconds. After finishing pressing, the press' surfaces are opened without pre-cooling. The collagen-based tube obtained is not transparent. It has an excellent mechanical stability, flexibility and good hemostatic properties. The central part - non adhesive material can be easily removed directly after manufacturing or later, i.e., directly before use.
After wetting, the tubular construct has an excellent mechanical stability and flexibility. It can be used for guiding tissue reconstruction, i.e. for reconstruction of tubular organs or nerves.

### Example 7

### Manufacture of a novel multilayer collagen-based material in form of tubes or channels in which the lumen is filled by the core made of an other collagen material of different porosity.

The product is made from freeze-dried and pre-pressed collagen membrane(s) and from a freeze-dried non-pre-pressed collagen sponge of different porosity in a similar way as described in Example 6. The not pre-pressed sponge is covered from all sides by pre-pressed collagen membrane(s) and treated simultaneously with heat and pressure to form a multilayer construct. A continuous heat and pressure, 100°C and 25 kg/cm², respectively, is applied to a period of sponges for 10 seconds. After finishing pressing, the press surfaces are opened without pre-cooling. The collagen-based tube obtained is not transparent. It has an excellent mechanical stability, flexibility and good hemostatic properties. The final material creates a construct with a core of different porosity. The core will absorb fluids up to 20x its own weight. The core is protected by a low-swelling collagen membrane. This construct can be used i.e. for reconstruction of missing tissue incl. bones and nerves.

### Example 8

Manufacture of a novel multilayer collagen-based membrane-like material in form of tubes or channels in which the lumen is filled by a core of an other collagen material of different porosity and in with core has additional longitudinally oriented channels.
The final product is manufactured as in Example 7, but additional longitudinally oriented channels are created in the core of the tube by incorporation of wire(s) of various diameters into the core material prior to manufacturing such tubes (see Example 7).

## Claims

1. A multilayer biocompatible sheet material comprising a first and second layer comprising matrices of biocompatible collagen, which layers are physically adhered along at least a portion of a surface of each of said layers, wherein said material has sufficient flexibility to form tubes useful for tissue reconstruction, and wherein at least one said layer matrix is capable of absorbing sufficient fluids to form an expanded matrix capable of promoting cell growth.

2. A multilayer biocompatible sheet material according to claim 1 wherein said one said layer matrix is porous and capable of promoting formation of granulation tissue, angiogenesis and vascularization.

3. A sheet material according to claim 1 or 2 wherein said layers remain adhered to each other in contact with water, blood and tissue fluids.

4. A sheet material according to claims 1, 2 or 3 wherein said collagen of each layer exhibits the hemostatic properties of native collagen.

5. A sheet material according to any of the preceding claims having a wet tensile strength useful in surgical suturing.

6. A sheet material according to any of the preceding claims wherein said sheet material further comprises from one to three additional layers each comprising matrices of biocompatible collagen.

7. A sheet material according to any of the preceding claims 1 to 5 wherein said sheet material further comprises a third layer comprising a biocompatible synthetic polymer.

8. A sheet material according to any of the preceding claims wherein said first layer comprises said porous matrix that expands to absorb a fluid amount up to 10 times in its own weight.

9. A sheet material according to any of the preceding claims wherein said second layer expands to absorb a fluid amount up to 10 times in its own weight.

10. A sheet material according to any of the preceding claims 1 to 7 wherein said first layer expands to absorb a fluid amount up to about 20 times in its own weight.

11. A sheet material according to any of the preceding claims wherein said first and second layers comprise the same permeability and porosity.

12. A sheet material according to any of the preceding claims 1 to 10 wherein said first and second layers comprise different permeability and porosity.

13. A tubular article having an inner and outer surface, comprising a sheet material according to any of the preceding claims wherein said first layer comprises said inner surface.

14. A tubular article according to claim 13 wherein said outer surface comprises a second layer that is a low-swelling collagen membrane protecting the core comprising said first layer.

15. A tubular article according to claim 13 or 14 wherein said outer surface has a low porosity and low air and water permeability.

16. A sheet material according to any of the preceding claims 1 to 12 wherein each layer comprises a compressed sponge or transparent membrane of reconstituted collagen.

17. A process for the preparation of a multilayer biocompatible sheet material having sufficient flexibility to form tubes useful for tissue reconstruction, comprising
(a) contacting a surface of a first sheet of a porous matrix of biocompatible collagen with a surface of a second sheet of matrix of biocompatible collagen to form a double layer construct and
(b) applying mechanical pressure and elevated temperature simultaneously and uniformly along the entirety of said contacting surfaces of said construct for a time sufficient to adhere said layers but insufficient to denature or degrade said biocompatible collagen, thereby forming a first multilayer sheet

18. A process according to claim 17, wherein said pressure is about 0.1 to about 1000 kg/cm², said elevated temperature is about 50 to about 200 degrees C, and said time is about 0.1 to about 60 seconds.

19. A process according to claim 18 said pressure is about 25 kg/cm² and wherein said elevated temperature is about 100 decrees C.

20. A process according to claim 18 wherein said time is about 10 seconds.

21. A process according to claim 17 wherein said biocompatible collagen matrices are sponges or transparent membranes and are formed from dispersions or suspensions of biocompatible collagen.

22. A process according to claim 17, further comprising aligning one or more additional layers of biocompatible collagen matrix with said first multilayer sheet forming a second construct, and applying mechanical pressure and elevated temperature simultaneously and uniformly along the entirety of said contacting surfaces of said second construct for a time sufficient to adhere said layers but insufficient to denature or degrade said biocompatible collagen.

23. A multilayer biocompatible sheet material having sufficient flexibility to form tubes useful for tissue reconstruction prepared according to the process of claim 17.

24. A multilayer material according to claim 1, wherein said layers comprise a sponge or membrane prepared from a dispersion or suspension containing 0.5 to 5 weight/volume % of collagen.

25. A multilayer material according to claim 24, wherein said layer has been previously compressed with simultaneous heat and pressure.

26. A multilayer material according to claim 1 further comprising biologically active substances, selected from the group consisting of hemostatic agents, growth factors, cytokines, hormones, antibiotics, anti-inflammatory agents.

## Patentansprüche

1. Biokompatibles Mehrschichtlagenmaterial, umfassend eine erste und zweite Schicht, die Matrizen von biokompatiblem Kollagen aufweisen, wobei die Schichten entlang mindestens eines Abschnitts einer Oberfläche jeder der Schichten physikalisch anhaften, worin das Material ausreichende Flexibilität aufweist, um für Geweberekonstruktion verwendbare Röhren zu bilden, und worin mindestens eine der Schichtmatrizen in der Lage ist, ausreichend Flüssigkeit zu absorbieren, um eine aufgeweitete Matrix zu bilden, die in der Lage ist, Zellwachstum zu fördern.

2. Biokompatibles Mehrschichtlagenmaterial nach Anspruch 1, worin die eine der Schichtmatrizen porös ist und in der Lage ist, die Bildung von Granulationsgewebe, Angiogenese und Vaskularisation zu fördern.

3. Lagenmaterial nach Anspruch 1 oder 2, worin die Schichten in Kontakt mit Wasser, Blut und Gewebeflüssigkeiten miteinander verbunden bleiben.

4. Lagenmaterial nach den Ansprüchen 1, 2 oder 3, worin das Kollagen jeder Schicht die hämostatischen Eigenschaften von natürlichem Kollagen zeigt.

5. Lagenmaterial nach irgendeinem der vorangehenden Ansprüche mit einer beim chirurgischen Nähen brauchbaren Naßreißfestigkeit.

6. Lagenmaterial nach irgendeinem der vorangehenden Ansprüche, worin das Lagenmaterial weiterhin 1 bis 3 zusätzliche Schichten, die jeweils Matrizen von biokompatiblem Kollagen aufweisen, umfaßt.

7. Lagenmaterial nach irgendeinem der vorangehenden Ansprüche 1 bis 5, worin das Lagenmaterial weiterhin eine dritte Schicht aufweist, die ein biokompatibles synthetisches Polymer umfaßt.

8. Lagenmaterial nach irgendeinem der vorangehenden Ansprüche, worin die erste Schicht die poröse Matrix umfaßt, die sich ausweitet, um eine Flüssigkeitsmenge bis zum Zehnfachen ihres Eigengewichts zu absorbieren.

9. Lagenmaterial nach irgendeinem der vorangehenden Ansprüche, worin die zweite Schicht sich ausweitet, um eine Flüssigkeitsmenge bis zum Zehnfachen ihres Eigengewicht zu absorbieren.

10. Lagenmaterial nach irgendeinem der vorangehenden Ansprüche 1 bis 7, worin die erste Schicht sich ausweitet, um eine Flüssigkeitsmenge bis zum etwa Zwanzigfachen ihres Eigengewichts zu absorbieren.

11. Lagenmaterial nach irgendeinem der vorangehenden Ansprüche, worin die ersten und zweiten Schichten dieselbe Permeabilität und Porosität aufweisen.

12. Lagenmaterial nach irgendeinem der vorangehenden Ansprüche 1 bis 10, worin die erste und zweite Schicht verschiedene Permeabilität und Porosität aufweisen.

13. Röhrenförmiger Gegenstand mit einer inneren und äußeren Oberfläche, umfassend ein Lagenmaterial nach irgendeinem der vorangehenden Ansprüche, wobei die erste Schicht die innere Oberfläche aufweist.

14. Röhrenförmiger Gegenstand nach Anspruch 13, worin die äußere Oberfläche eine zweite Schicht aufweist, die eine gering aufquellende Kollagenmembran ist, die den Kern, der die erste Schicht umfaßt, schützt.

15. Röhrenförmiger Gegenstand nach Anspruch 13 oder 14, worin die äußere Oberfläche eine geringe Porosität und eine geringe Luft- und Wasserpermeabilität aufweist.

16. Lagenmaterial nach irgendeinem der vorangehenden Ansprüche 1 bis 12, worin jede Schicht einen komprimierten Schwamm oder eine transparente Membran aus rekonstituiertem Kollagen aufweist.

17. Verfahren zur Herstellung eines biokompatiblen Mehrschichtlagenmaterials mit ausreichender Flexibilität um für Geweberekonstruktion verwendbare Röhren zu bilden, umfassend
(a) in Kontakt bringen einer Oberfläche einer ersten Lage einer porösen Matrix von biokompatiblem Kollagen mit einer Oberfläche einer zweiten Lage einer Matrix von biokompatiblem Kollagen, um ein Doppelschichtkonstrukt zu bilden und
(b) gleichzeitiges und gleichmäßiges Einsetzen von mechanischem Druck und erhöhter Temperatur entlang der Gesamtheit der kontaktierenden Oberflächen des Konstrukts für eine Zeit, die ausreichend ist, die Schichten anzuhaften, aber nicht ausreicht, das biokompatible Kollagen zu denaturieren oder abzubauen, wodurch eine erste Mehrschichtlage gebildet wird.

18. Verfahren nach Anspruch 17, worin der Druck etwa 0,1 bis etwa 1.000 kg/cm² beträgt, die erhöhte Temperatur etwa 50 bis etwa 200°C beträgt und die Zeit etwa 0,1 bis etwa 60 Sekunden beträgt.

19. Verfahren nach Anspruch 18, worin der Druck etwa 25 kg/cm² beträgt und worin die erhöhte Temperatur etwa 100°C beträgt.

20. Verfahren nach Anspruch 18, worin die Zeit etwa 10 Sekunden beträgt.

21. Verfahren nach Anspruch 17, worin die biokompatiblen Kollagenmatrizen Schwämme oder transparente Membranen darstellen, und aus Dispersionen oder Suspensionen von biokompatiblem Kollagen gebildet werden.

22. Verfahren nach Anspruch 17, weiter umfassend Bilden eines zweiten Konstrukts durch Ausrichten von ein oder mehreren zusätzlichen Schichten einer biokompatiblen Kollagenmatrix mit der ersten Mehrschichtlage, und gleichzeitiges und gleichmäßiges Einsetzen von mechanischem Druck und erhöhter Temperatur entlang der Gesamtheit der kontaktierenden Oberflächen des zweiten Konstrukts für eine Zeit, die ausreicht, die Schichten anzuhaften, aber nicht ausreicht, das biokompatible Kollagen zu denaturieren oder abzubauen.

23. Biokompatibles Mehrschichtlagenmaterial mit ausreichender Flexibilität, um für Geweberekonstruktion verwendbare Röhren zu bilden, hergestellt nach dem Verfahren von Anspruch 17.

24. Mehrschichtmaterial nach Anspruch 1, worin die Schichten einen Schwamm oder eine Membran aufweisen, hergestellt aus einer Dispersion oder Suspension, enthaltend 0,5 bis 5 Gew./Vol.-% Kollagen.

25. Mehrschichtmaterial nach Anspruch 24, worin die Schicht unter gleichzeitiger Wärme und Druck zuvor komprimiert wurde.

26. Mehrschichtmaterial nach Anspruch 1, weiterhin umfassend biologisch aktive Substanzen, ausgewählt aus der Gruppe, bestehend aus hämostatischen Mitteln, Wachstumsfaktoren, Cytokinen, Hormonen, Antibiotika und entzündungshemmenden Mitteln.

## Revendications

1. Matériau en feuilles biocompatible multicouche comprenant une première et une seconde couches comprenant des matrices de collagène biocompatible, lesquelles couches sont physiquement collées le long d'au moins une partie d'une surface de chacune desdites couches, dans lequel ledit matériau a une flexibilité suffisante pour former des tubes utiles pour une reconstruction de tissu, et dans lequel au moins une de ladite matrice en couches est capable d'absorber suffisamment de liquides pour former une matrice expansée capable de promouvoir une croissance cellulaire.

2. Matériau en feuilles biocompatible multicouche selon la revendication 1, dans lequel ladite une de ladite matrice en couches est poreuse et capable de promouvoir la formation d'un tissu de granulation, une angiogenèse et une vascularisation.

3. Matériau en feuilles selon la revendication 1 ou 2, dans lequel lesdites couches restent collées l'une à l'autre au contact avec l'eau, le sang et les liquides interstitiels.

4. Matériau en feuilles selon les revendications 1, 2 ou 3, dans lequel ledit collagène de chaque couche montre les propriétés hémostatiques du collagène natif.

5. Matériau en feuilles selon l'une quelconque des revendications précédentes ayant une résistance à la traction à l'état humide utile dans le suturage chirurgical.

6. Matériau en feuilles selon l'une quelconque des revendications précédentes, dans lequel ledit matériau en feuilles comprend en outre de une à trois couches supplémentaires comprenant chacune des matrices de collagène biocompatible.

7. Matériau en feuilles selon l'une quelconque des revendications précédentes 1 à 5, dans lequel ledit matériau en feuilles comprend en outre une troisième couche comprenant un polymère synthétique biocompatible.

8. Matériau en feuilles selon l'une quelconque des revendications précédentes, dans lequel ladite première couche comprend ladite matrice poreuse qui se dilate pour absorber une quantité de liquide de jusqu'à 10 fois son propre poids.

9. Matériau en feuilles selon l'une quelconque des revendications précédentes, dans lequel ladite seconde couche se dilate pour absorber une quantité de liquide de jusqu'à 10 fois son propre poids.

10. Matériau en feuilles selon l'une quelconque des revendications précédentes 1 à 7, dans lequel ladite première couche se dilate pour absorber une quantité de liquide de jusqu'à 20 fois son propre poids.

11. Matériau en feuilles selon l'une quelconque des revendications précédentes, dans lequel lesdites première et seconde couches comprennent les mêmes perméabilité et porosité.

12. Matériau en feuilles selon l'une quelconque des revendications précédentes 1 à 10, dans lequel lesdites première et seconde couches comprennent des perméabilité et porosité différentes.

13. Article tubulaire ayant une surface interne et externe, comprenant un matériau en feuilles selon l'une quelconque des revendications précédentes, dans lequel ladite première couche comprend ladite surface interne.

14. Article tubulaire selon la revendication 13, dans lequel ladite surface externe comprend une seconde couche qui est une membrane de collagène à faible gonflement protégeant le coeur comprenant ladite première couche.

15. Article tubulaire selon la revendication 13 ou 14, dans lequel ladite surface externe a une porosité faible et une perméabilité à l'air et à l'eau faible.

16. Matériau en feuilles selon l'une quelconque des revendications précédentes 1 à 12, dans lequel chaque couche comprend une membrane transparente de collagène reconstitué ou une éponge comprimée de collagène reconstitué.

17. Procédé de préparation d'un matériau en feuilles biocompatible multicouche ayant une flexibilité suffisante pour former des tubes utiles pour une reconstruction de tissu, comprenant
(a) mettre en contact la surface d'une première feuille d'une matrice poreuse de collagène biocompatible avec une surface d'une seconde feuille de matrice de collagène biocompatible pour former une construction à double couche, et
(b) appliquer une pression mécanique et une température élevée simultanément et uniformément le long de la totalité desdites surfaces en contact de ladite construction pendant un temps suffisant pour coller lesdites couches mais insuffisant pour dénaturer ou dégrader ledit collagène biocompatible, en formant par ce moyen une première feuille multicouche.

18. Procédé selon la revendication 17, dans lequel ladite pression est d'environ 0,1 à environ 1000 kg/cm², ladite température élevée est d'environ 50 à environ 200 degrés C, et ledit temps est d'environ 0,1 à environ 60 secondes.

19. Procédé selon la revendication 18, dans lequel ladite pression est d'environ 25 kg/cm² et dans lequel ladite température élevée est d'environ 100 degrés C.

20. Procédé selon la revendication 18, dans lequel ledit temps est d'environ 10 secondes.

21. Procédé selon la revendication 17, dans lequel lesdites matrices de collagène biocompatibles sont des éponges ou des membranes transparentes et sont formées à partir de dispersions ou de suspensions de collagène biocompatible.

22. Procédé selon la revendication 17, comprenant en outre l'alignement d'une ou de plusieurs couches supplémentaires d'une matrice de collagène biocompatible avec ladite première feuille multicouche formant une seconde construction, et l'application d'une pression mécanique et d'une température élevée simultanément et uniformément le long de la totalité desdites surfaces en contact de ladite seconde construction pendant un temps suffisant pour coller lesdites couches mais insuffisant pour dénaturer ou dégrader ledit collagène biocompatible.

23. Matériau en feuilles biocompatible multicouche ayant une flexibilité suffisante pour former des tubes utiles pour une reconstruction de tissu préparé selon le procédé de la revendication 17.

24. Matériau multicouche selon la revendication 1, dans lequel lesdites couches comprennent une éponge ou une membrane préparée à partir d'une dispersion ou d'une suspension contenant de 0,5 à 5 % en poids/volume de collagène.

25. Matériau multicouche selon la revendication 24, dans lequel ladite couche a été précédemment comprimée simultanément par la chaleur et la pression.

26. Matériau multicouche selon la revendication 1, comprenant en outre des substances biologiquement actives, sélectionnées dans le groupe consistant en agents hémostatiques, facteurs de croissance, cytokines, hormones, antibiotiques, agents anti-inflammatoires.
